# EUROPEAN PATENT SPECIFICATION

(11) **EP 2 149 343 B1**
(45) Date of publication and mention of the grant of the patent: **08.07.2015**
(21) Application number: 09166708.9
(22) Date of filing: 29.07.2009
(51) Int. Cl.: A61B 18/12, A61N 5/02

(54) **Tissue ablation system with phase-controlled channels**
Gewebeablationssystem mit phasengesteuerten Kanälen
Système d'ablation de tissus doté de voies contrôlées par phase

(30) Priority: 29.07.2008 US 181504
(43) Date of publication of application: 03.02.2010
(73) Proprietor: Covidien LP, Mansfield, MA 02048 (US)
(72) Inventor: Brannan, Joseph D., Erie, CO 80516 (US); Behzad, Ghorbani-Elizeh, Boulder, CO 80301 (US)
(74) Representative: Hoffmann Eitle

(56) References cited:
- WO-A-2008/068485
- US-A- 4 397 313
- US-A- 4 462 412
- US-A- 4 572 190
- US-A- 4 798 215
- US-A- 4 815 479
- US-A- 5 097 844
- US-A1- 2007 118 193
- US-B1- 6 208 903

## Description

### BACKGROUND

### 1. Technical Field

The present disclosure relates to apparatus and methods for providing energy to tissue and, more particularly, to devices and electromagnetic radiation delivery procedures utilizing ablation probes and methods of controlling the delivery of electromagnetic radiation to tissue.

### 2. Discussion of Related Art

Treatment of certain diseases requires destruction of malignant tumors. Electromagnetic radiation can be used to heat and destroy tumor cells. Treatment may involve inserting ablation probes into tissues where cancerous tumors have been identified. Once the probes are positioned, electromagnetic energy is passed through the probes into surrounding tissue.

In the treatment of diseases such as cancer, certain types of cancer cells have been found to denature at elevated temperatures that are slightly lower than temperatures normally injurious to healthy cells. Known treatment methods, such as hyperthermia therapy, use electromagnetic radiation to heat diseased cells to temperatures above 41° C while maintaining adjacent healthy cells below the temperature at which irreversible cell destruction occurs. These methods involve applying electromagnetic radiation to heat, ablate and/or coagulate tissue. Microwave energy is sometimes utilized to perform these methods. Other procedures utilizing electromagnetic radiation to heat tissue also include coagulation, cutting and/or ablation of tissue.

Electrosurgical devices utilizing electromagnetic radiation have been developed for a variety of uses and applications. A number of devices are available that can be used to provide high bursts of energy for short periods of time to achieve cutting and coagulative effects on various tissues. There are a number of different types of apparatus that can be used to perform ablation procedures. Typically, microwave apparatus for use in ablation procedures include a microwave generator, which functions as an energy source, and a microwave surgical instrument having an antenna assembly for directing the energy to the target tissue. The microwave generator and surgical instrument are typically operatively coupled by a cable assembly having a plurality of conductors for transmitting microwave energy from the generator to the instrument, and for communicating control, feedback and identification signals between the instrument and the generator.

Microwave energy is typically applied via antenna assemblies that can penetrate tissue. Several types of antenna assemblies are known, such as monopole and dipole antenna assemblies. In monopole and dipole antenna assemblies, microwave energy generally radiates perpendicularly away from the axis of the conductor. A monopole antenna assembly includes a single, elongated conductor that transmits microwave energy. A typical dipole antenna assembly has two elongated conductors, which are linearly aligned and positioned end-to-end relative to one another with an electrical insulator placed therebetween. Each conductor may be about 1/4 of the length of a wavelength of the microwave energy, making the aggregate length of the two conductors about 1/2 of the wavelength of the supplied microwave energy. During certain procedures, it can be difficult to assess the extent to which the microwave energy will radiate into the surrounding tissue, making it difficult to determine the area or volume of surrounding tissue that will be ablated.

Further, US-A-4,798,215 is concerned with a hyperthermia apparatus.

### SUMMARY

The present disclosure relates to a system for applying energy to tissue via a plurality of channels. The system includes a controller adapted to connect to an energy source, wherein the controller is configured to control a phase relationship between electrical signals in each channel, and a number of energy delivery devices, each energy delivery device operatively coupled to the controller via a corresponding one of the channels.

According to another exemplary embodiment of the present disclosure, a system for applying energy to tissue via N channels, where N is an integer greater than 1, includes at least one energy source to generate electrical signals for transmission on the N channels, a phase monitoring and adjusting module coupled to the at least one energy source, the phase monitoring and adjusting module including N outputs and N phase shifters to adjust a phase of an electrical signal on each of the N channels with respect to the other N-1 channels to a predetermined phase relationship, and N energy delivery devices, each respectively operably coupled to a corresponding one of the N outputs of the phase monitoring and adjusting module via a corresponding one of the N channels.

According to yet another exemplary embodiment of the present disclosure, a method for directing energy to a target tissue is disclosed and includes the steps of: positioning a plurality of energy delivery devices into a portion of the target tissue; transmitting a plurality of electrical signals on a plurality of channels to the energy delivery devices in a set of phase relationships between the electrical signals; and applying energy from an energy-directing element of each energy delivery device to the target tissue.

The invention is defined in claim 1. The dependent claims define further embodiments of the invention.

Objects and features of the presently disclosed tissue ablation systems with phase-controlled channels will become readily apparent to those of ordinary skill in the art when descriptions of various embodiments thereof are read with reference to the accompanying drawings.

### BRIEF DESCRIPTION OF THE DRAWINGS

FIG. 1 is a schematic diagram of an electrosurgical system for treating tissue, according to an exemplary embodiment of the present disclosure;
FIG. 2 is a schematic diagram of an electrosurgical system for treating tissue, according to an exemplary embodiment of the present disclosure;
FIG. 3 is a schematic diagram of an electrosurgical system for treating tissue, according to an exemplary embodiment of the present disclosure;
FIG. 4 is a schematic diagram of an electrosurgical system for treating tissue, according to an exemplary embodiment of the present disclosure;
FIG. 5 is a schematically-illustrated representation of simulation results showing power absorption and two wire standing wave behavior between probes, according to an exemplary embodiment of the present disclosure;
FIG. 6 is a schematically-illustrated representation of a biological tissue image showing thermal effects of out-of-phase excitation between and up toward the surface of antenna shafts, according to an exemplary embodiment of the present disclosure;
FIG. 7 is a schematically-illustrated representation of a biological tissue image showing thermal effects of in-phase excitation between and up toward the surface of antenna shafts, according to an exemplary embodiment of the present disclosure; and
FIG. 8 is a flowchart illustrating a method for directing energy to a target tissue, according to an exemplary embodiment of the present disclosure.

### DESCRIPTION OF EXEMPLARY EMBODIMENTS

Hereinafter, exemplary embodiments the presently disclosed tissue ablation systems with phase-controlled channels are described with reference to the accompanying drawings. Like reference numerals may refer to similar or identical elements throughout the description of the figures. As used herein, the term "microwave" generally refers to electromagnetic waves in the frequency range of 300 megahertz (MHz) (3 x 10⁸ cycles/second) to 300 gigahertz (GHz) (3 x 10¹¹ cycles/second). As used herein, the phrase "transmission line" generally refers to any transmission medium that can be used for the propagation of signals from one point to another.

Various exemplary embodiments of the present disclosure provide electrosurgical systems for treating tissue and methods of controlling the delivery of electromagnetic radiation to tissue. Exemplary embodiments may be implemented using electromagnetic radiation at microwave frequencies or at other frequencies. Electrosurgical systems for treating tissue, according to various exemplary embodiments of the present disclosure, deliver phase-controlled microwave power to a plurality of electrosurgical devices while maintaining a phase balance of <+/-45 degrees. Electrosurgical devices, such as ablation probes, for implementing exemplary embodiments of the present disclosure may be inserted directly into tissue, inserted through a lumen, such as a vein, needle or catheter, placed into the body during surgery by a clinician or positioned in the body by other suitable methods or means known in the art. Although various exemplary methods described hereinbelow are targeted toward microwave ablation and the complete destruction of target tissue, it is to be understood that exemplary methods of controlling the delivery of electromagnetic radiation may be used with other therapies in which the target tissue is partially destroyed or damaged, such as to prevent the conduction of electrical impulses within heart tissue.

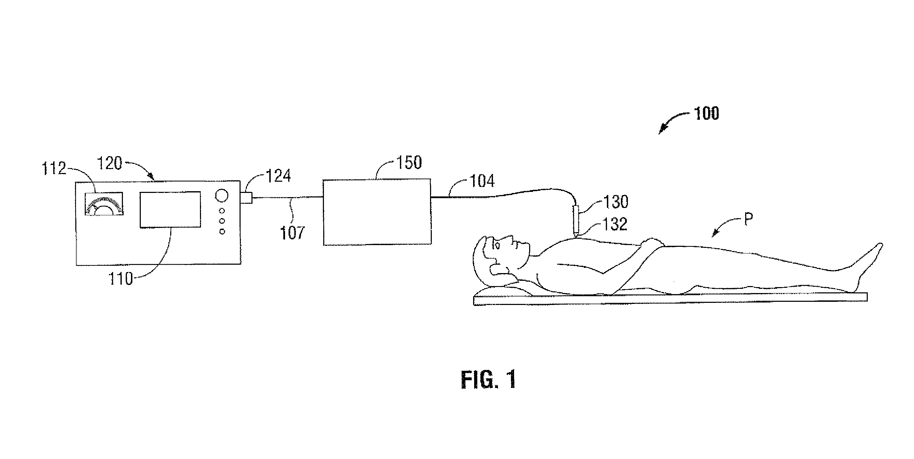

FIG. 1 is a schematic diagram of an electrosurgical system for treating tissue, according to an exemplary embodiment of the present disclosure. Referring to FIG. 1, the electrosurgical system 100 includes an electrosurgical generator 120 for generating an output signal, a controller 150 coupled to the electrosurgical generator 120, and an electrosurgical instrument or device 130 coupled to the controller 150. The controller 150 is coupled to a transmission line 107 that electrically connects the controller 150 to an output 124 on the electrosurgical generator 120. The device 130 includes an antenna assembly 132 for delivery of electromagnetic radiation, coupled to a transmission line 104 that electrically connects the antenna assembly 132 to the controller 150. Although not shown as such in FIG. 1, device 130 may include a plurality of antenna assemblies.

The electrosurgical generator 120 includes a graphical user interface 110 and a dial indicator 112. The electrosurgical generator 120 may include other input or output devices such as knobs, dials, switches, buttons, displays and the like for control, indication and/or operation. The electrosurgical generator 120 may be capable of generating a plurality of output signals of various frequencies that are input to the controller 150. In an exemplary embodiment of the present disclosure, the electrosurgical generator 120 generates a plurality of microwave signals at substantially the same frequency. The electrosurgical generator 120 may include a control unit (not shown) that controls operations of the electrosurgical generator 120, such as time of operation, power output and/or the mode of electrosurgical operation, which may have been selected by the clinician.

The electrosurgical system 100 may include a footswitch (not shown) coupled to the electrosurgical generator 120. When actuated, the footswitch causes the electrosurgical generator 120 to generate microwave energy. The device 130 may include knobs, dials, switches, buttons or the like (not shown) to communicate to the electrosurgical generator 102 to adjust or select from a number of configuration options for delivering energy. Utilizing knobs, dials, switches or buttons on the device 130 and/or a footswitch enables the clinician to activate the electrosurgical generator 120 to energize the device 130 while remaining near the patient P regardless of the location of the electrosurgical generator 102.

Although not shown as such in FIG. 1, electrosurgical system 100 may include a plurality of channels defined by a plurality of electrosurgical devices and a plurality of transmission lines that electrically connect the electrosurgical devices to the controller 150. In an exemplary embodiment of the present disclosure, the controller 150 is capable of monitoring the phase of each channel and adjusting the phase of the signal in each channel with respect to the other channel(s) to a predetermined phase relationship. The controller 150 provides a plurality of signals to the device 130 in a set of phase relationships between the signals. Although the controller 150 is illustrated as a standalone module in FIG. 1, it is to be understood that the controller 150 may be integrated fully or partially into the electrosurgical generator 120, the device 130 and/or other devices.

The antenna assembly 132 includes multiple antennas and/or multiple antenna elements, each driven by an output signal of the controller 150. The antenna assembly 132 may also include multiple antenna circuits, each driven by an output signal of the controller 150.

In an exemplary embodiment of the present disclosure, the antenna assembly 132 is typically a microwave antenna configured to allow direct insertion or penetration into tissue of the patient P. The antenna assembly 132 may be axially rigid to allow for tissue penetration. The antenna assembly 132 is sufficiently small in diameter to be minimally invasive of the body, which may reduce the preparation of the patient P as might be required for more invasive penetration of the body. The antenna assembly 132 is inserted directly into tissue, inserted through a lumen, such as, for example, a vein, needle or catheter, placed into the body during surgery by a clinician, or positioned in the body by other suitable methods or means known in the art.

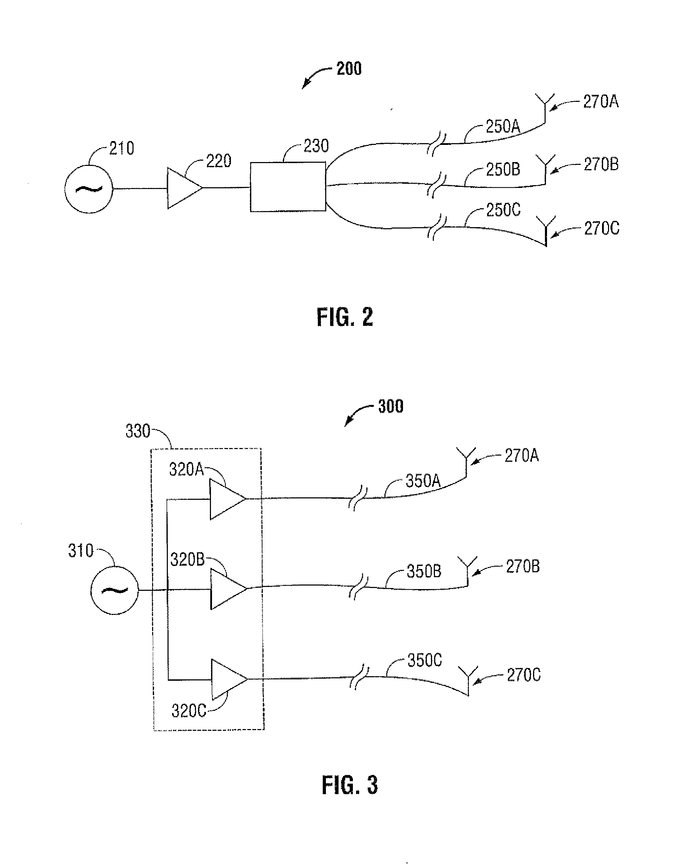

FIG. 2 is a schematic diagram of an electrosurgical system for treating tissue, according to another exemplary embodiment of the present disclosure. Referring to FIG. 2, the electrosurgical system 200 includes a microwave signal source 210 providing a microwave frequency output signal to a microwave amplifier unit 220, a phase-balanced microwave power splitter 230 coupled to the microwave amplifier unit 220, and a first, a second and a third microwave ablation antenna assembly 270A, 2708 and 270C, each coupled to the phase-balanced microwave power splitter 230. The microwave signal source 210 is capable of generating a plurality of output signals of various frequencies that are input to the microwave amplifier unit 220. The microwave amplifier unit 220 may have any suitable input power and output power.

In the electrosurgical system 200, a first transmission line 250A electrically connects the first antenna assembly 270A to the phase-balanced microwave power splitter 230, defining a first channel; a second transmission line 250B electrically connects the second antenna assembly 270B to the phase-balanced microwave power splitter 230, defining a second channel; and a third transmission line 250C electrically connects the third antenna assembly 270C to the phase-balanced microwave power splitter 230, defining a third channel. The first, second and third transmission lines 250A, 250B and 250C may each include one or more electrically conductive elements, such as electrically conductive wires.

In an exemplary embodiment, the first, second and third transmission lines 250A, 250B and 250C each have substantially the same length, which preserves the phase relationship between the electrical signals in each channel of the electrosurgical system 200. It is to be understood that "length" may refer to electrical length or physical length. In general, electrical length is an expression of the length of a transmission medium in terms of the wavelength of a signal propagating within the medium. Electrical length is normally expressed in terms of wavelength, radius or degrees. For example, electrical length may be expressed as a multiple or sub-multiple of the wavelength of an electromagnetic wave or electrical signal propagating within a transmission medium. The wavelength may be expressed in radians or in artificial units of angular measure, such as degrees. The phase-balanced microwave power splitter 230 may be implemented by any suitable power divider that provides equal power split at all output ports while substantially maintaining phase. For example, the phase-balanced microwave power splitter 230 may be implemented using a 3-way power divider that provides equal power split at all output ports while maintaining a phase balance of <+/-45 degrees. The phase-balanced microwave power splitter 230 may be implemented by any suitable power divider that provides equal power split at all output ports while substantially maintaining phase and amplitude balance. For example, in one instance, the phase-balanced microwave power splitter 230 implements using a 3-way power divider that provides equal power split at all output ports while maintaining a phase balance of <+/-10 degrees and amplitude balance of <1.5 dB.

Each antenna assembly 270A, 270B and 270C typically includes a plurality of electrodes disposed on a rigid or bendable needle or needle-like structure. The antenna assemblies 270A, 270B and 270C are positioned substantially parallel to each other, for example, spaced about 5 millimeters (mm) apart, and inserted directly into tissue or placed into the body during surgery by a clinician, or positioned in the body by other suitable methods. Although the electrosurgical system 200 illustrated in FIG. 2 includes three microwave ablation antenna assemblies 270A, 270B and 270C, it is to be understood that any "N" number of antenna assemblies may be utilized and that phase-balanced microwave power splitter 230 may be implemented by any suitable power divider that divides or splits a microwave input signal into "N" number of output signals of equal power while substantially maintaining phase and amplitude balance.

The electrosurgical system 200 delivers phase-controlled microwave power to each antenna assembly 270A, 270B and 270C of the three-channel system. The electrosurgical system 200 delivers substantially in-phase microwave power to each antenna assembly 270A, 270B and 270C, which may result in a more efficient ablating tool than out-of-phase probes. By controlling the phase of ablation probes with respect to each other, according to exemplary embodiments of the present disclosure, a desired effect on tissue between the probes is produced. In a resection procedure where a long thin ablation line is desired, probes that are 180 degrees out of phase with respect to each other produce a desired effect on tissue. In ablation procedures using in-phase probes, according to various exemplary embodiments of the present disclosure, there may be a reduction in energy that might otherwise move between the antenna shafts toward the surface with out-of-phase probes.

In an exemplary embodiment, the electrosurgical system 200 delivers phase-controlled microwave power to each antenna assembly 270A, 270B and 270C while maintaining a phase balance of <+/-45 degrees. The electrosurgical system 200 is implemented with operating frequencies in the range of about 915 MHz to about 5 GHz, which may be useful in performing ablation procedures and/or other procedures. It is to be understood that the electrosurgical system 200 may be implemented with any appropriate range of operating frequencies.

FIG. 3 is a schematic diagram of an electrosurgical system for treating tissue, according to an exemplary embodiment of the present disclosure. Referring to FIG. 3, the electrosurgical system 300 includes a microwave signal source 310 providing a microwave frequency output signal to a controller 330, and a first, a second and a third microwave ablation antenna assembly 270A, 2708 and 270C, each coupled to the controller 330. The microwave signal source 310 is capable of generating a plurality of output signals of various frequencies that are input to the controller 330.

The controller 330 includes a first, a second and a third microwave amplifier 320A, 320B and 320C that are phase-balanced with respect to one another. The first, second and third phase-balanced microwave amplifiers 320A, 320B and 320C each deliver equal power while maintaining a phase balance of <+1-10 degrees and amplitude balance of <1.5 dB. In an exemplary embodiment, the first, second and third phase-balanced microwave amplifiers 320A, 320B and 320C each deliver phase-controlled microwave power to the respective antenna assemblies 270A, 270B and 270C while maintaining a phase balance of <+/-45 degrees. The first, second and third phase-balanced microwave amplifiers 320A, 320B and 320C may have any suitable input power and output power.

In the electrosurgical system 300, a first transmission line 350A electrically connects the first antenna assembly 270A to the first phase-balanced microwave amplifier 320A, defining a first channel; a second transmission line 350B electrically connects the second antenna assembly 270B to the second phase-balanced microwave amplifier 320B, defining a second channel; and a third transmission line 350C electrically connects the third antenna assembly 270C to the third phase-balanced microwave amplifier 320C, defining a third channel. The first, second and third transmission lines 350A, 350B and 350C each include one or more electrically conductive elements, such as electrically conductive wires. In an exemplary embodiment, the first, second and third transmission lines 350A, 350B and 350C each have substantially the same length, which preserves the phase relationship between electrical signals in each channel of the electrosurgical system 300.

Although the electrosurgical system 300 illustrated in FIG. 3 includes three microwave ablation antenna assemblies 270A, 270B and 270C and three phase-balanced microwave amplifiers 320A, 320B and 320C, it is to be understood that any N number of antenna assemblies and any N number of phase-balanced microwave amplifiers may be utilized.

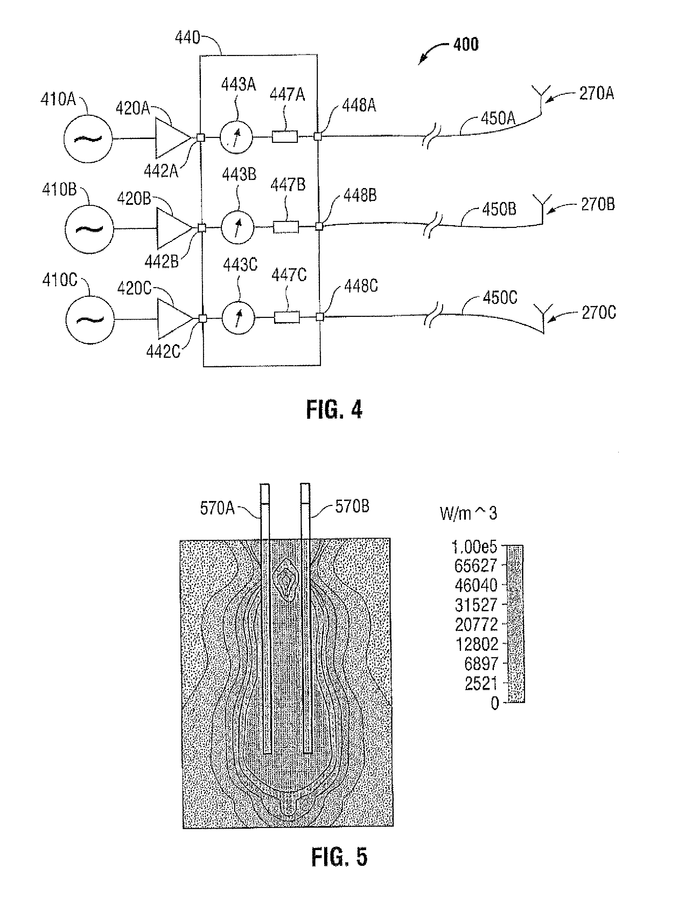

FIG. 4 is a schematic diagram of an electrosurgical system for treating tissue, according to another exemplary embodiment of the present disclosure. Referring to FIG. 4, the electrosurgical system 400 illustrated is a three-channel system that includes a first, a second and a third microwave signal source 410A, 410B and 410C, a first, a second and a third microwave amplifier 420A, 4208 and 420C, a controller 440 that includes three inputs 442A, 442B and 442C and three outputs 448A, 448B and 448C, and a first, a second and a third microwave ablation antenna assembly 270A, 270B and 270C.

The first, second and third microwave signal sources 410A, 410B and 410C provide microwave frequency output signals to the first, second and third amplifiers 420A, 420B and 420C, respectively. The first microwave amplifier 420A provides an output signal through an output terminal that is electrically coupled to the first input 442A of the controller 440; the second microwave amplifier 420B provides an output signal through an output terminal that is electrically coupled to the second input 442B of the controller 440; and the third microwave amplifier 420C provides an output signal through an output terminal that is electrically coupled to the third input 442C of the controller 440. The first, second and third amplifiers 420A, 420B and 420C each have any suitable input power and output power. In an exemplary embodiment, the first, second and third amplifiers 420A, 420B and 420C may be phase-balanced with respect to one another and, in such case, are arranged between the controller 440 and the first, second and third microwave ablation antenna assemblies 270A, 270B and 270C.

Although the first, second and third amplifiers 420A, 420B and 420C are illustrated as standalone modules in FIG. 4, it is to be understood that one or more of the amplifiers may be integrated fully or partially into the controller 440. The electrosurgical system 400 may be implemented without the first, second and third amplifiers 420A, 420B and 420C, or with any combination thereof.

The controller 440 includes a first, a second and a third phase shifter 443A, 443B and 443C, and a first, a second and a third phase monitor unit 447A, 447B and 447C. The first phase shifter 443A is electrically coupled between the first input 442A and the first phase monitor unit 447A; the second phase shifter 443B is electrically coupled between the second input 442B and the second phase monitor unit 4478; and the third phase shifter 443C is electrically coupled between the third input 442C and the third phase monitor unit 447C. The first phase monitor unit 447A is electrically coupled between the first phase shifter 443A and the output 448A; the second phase monitor unit 447B is electrically coupled between the second phase shifter 443B and the output 448B; and the third phase monitor unit 447C is electrically coupled between the third phase shifter 443C and the output 448C.

The controller 440 may include a number of processing units (not shown) coupled to the first, second and third phase monitor units 447A, 4478 and 447C for controlling output of one or more of the phase shifters 443A, 443B and 443C to provide a desired phase relationship of electrical signals in each channel of the electrosurgical system 400. The processing unit may include multiple processors and/or multicore CPUs and may include any type of processor capable of executing software, such as a microprocessor, digital signal processor, microcontroller, or the like.

The controller 440 may include one or more phase detectors (not shown) to compare the respective phases of electrical signals inputted through the inputs 442A, 442B and/or 442C. By comparing a reference signal, such as a clock signal, to a feedback signal using a phase detector, phase adjustments may be made based on the comparison of the electrical signals inputted, to set the phase relationship between electrical signals in each channel of the electrosurgical system 400.

In an exemplary embodiment, the controller 440 delivers phase-controlled microwave power through the outputs 448A, 448B and 448C to the antenna assemblies 270A, 270B and 270C, respectively irrespective of the individual phases of each of electrical signals inputted through the inputs 442A, 442B and/or 442C. As illustrated in FIG. 4, a first transmission line 450A electrically connects the first antenna assembly 270A to the output 448A of the controller 440, defining a first channel; a second transmission line 450B electrically connects the second antenna assembly 270B to the output 448B of the controller 440, defining a second channel; and a third transmission line 450C electrically connects the third antenna assembly 270C to the output 448C of the controller 440, defining a third channel. The first, second and third transmission lines 450A, 450B and 450C each include one or more electrically conductive elements, such as electrically conductive wires. In an exemplary embodiment, the first, second and third transmission lines 450A, 450B and 450C each have substantially the same length, which preserves the phase relationship between electrical signals in each channel of the electrosurgical system 400.

FIG. 5 is a schematically-illustrated representation of simulation results showing power absorption and two wire standing wave behavior between probes, according to an exemplary embodiment of the present disclosure. The illustrated results are based on a simulation which modeled parallel-arranged probes 570A and 570B spaced 5 mm apart and supplied with voltages out of phase with each other.

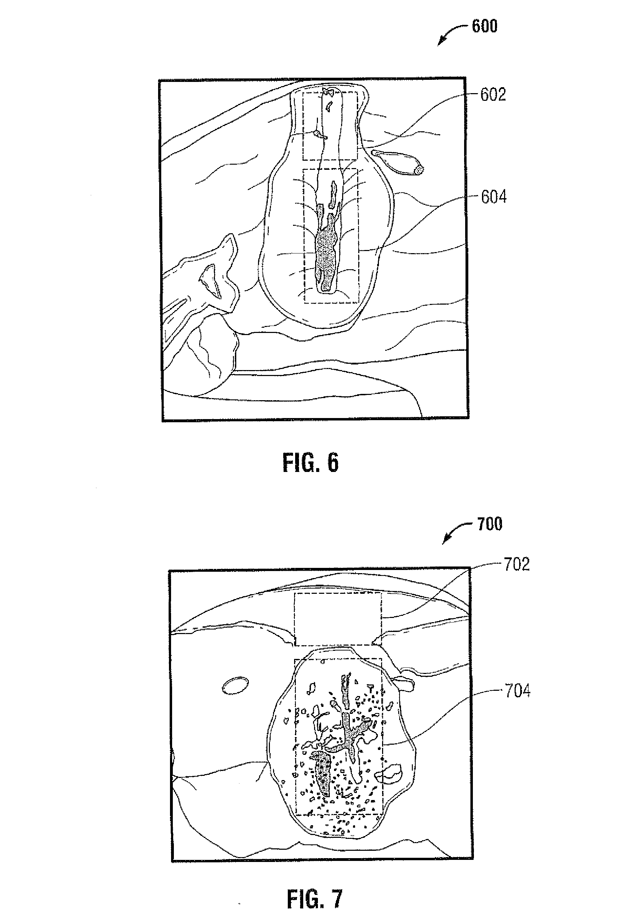

FIG. 6 is a schematically-illustrated representation of a biological tissue image showing thermal effects of out-of-phase excitation between and up toward the surface of antenna shafts, according to an exemplary embodiment of the present disclosure. Referring to FIG. 6, the tissue image 600 is divided into an upper region 602 and lower region 604. The lower region 604 is characterized by a burned area and surrounding ablation damaged tissue. In the tissue image 600, the ablation damaged tissue extends into the upper region 602.

FIG. 7 is a schematically-illustrated representation of a biological tissue image showing thermal effects of in-phase excitation between and up toward the surface of antenna shafts, according to an exemplary embodiment of the present disclosure. Referring to FIG. 7, the tissue image 700 is divided into an upper region 702 and a lower region 704. The lower region 704 is characterized by a burned area and surrounding ablation damaged tissue. In tissue image 600, the ablation damaged tissue does not extend into the upper region 702. Thus, thermal effects of in-phase excitation shown in the tissue image 700 are reduced toward the surface of antenna shafts (upper region 702), as compared to thermal effects of out-of-phase excitation shown in the upper region 602 of FIG. 6.

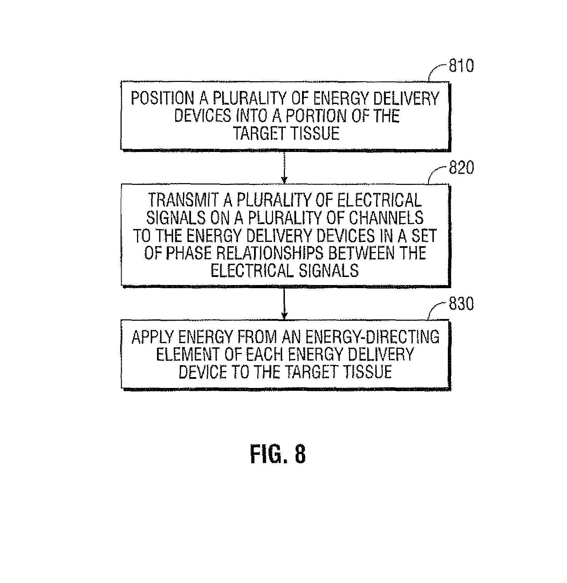

FIG. 8 is a flowchart illustrating a method for directing energy to a target tissue, according to an exemplary embodiment of the present disclosure. Referring to FIG. 8, in block 810, a plurality of energy delivery devices are positioned into a portion of the target tissue. The energy delivery devices may be implemented using any suitable electrosurgical instruments or devices, such as, for example, the device 130, according to exemplary embodiments of the present disclosure described in connection with FIG. 1.

The energy delivery devices are positioned into a portion of a target site on the tissue or adjacent to a portion of a target site on the tissue. The energy delivery devices are inserted directly into tissue, inserted through a lumen, such as a vein, needle or catheter, placed into the body during surgery by a clinician or positioned in the body by other suitable methods or means known in the art. The energy delivery devices include any suitable antenna assemblies for the delivery of electromagnetic radiation, such as, for example, the antenna assemblies 270A, 270B and 270C, according to exemplary embodiments of the present disclosure described in connection with FIG. 2.

In block 820, a plurality of electrical signals are transmitted on a plurality of channels to the energy delivery devices in a set of phase relationships between the electrical signals. For example, the electrical signals may be transmitted to the energy delivery devices from the controller 230, according to exemplary embodiments of the present disclosure described in connection with FIG. 2, the controller 330, according to exemplary embodiments of the present disclosure described in connection with FIG. 3, or the controller 440, according to exemplary embodiments of the present disclosure described in connection with FIG. 4. The set of phase relationships may be defined as a phase balance of <+/-45 degrees between the electrical signals on each channel.

In block 830, energy from an energy-directing element of each energy delivery device is applied to the target tissue. For example, the energy may be microwave energy.

## Claims

1. An electrosurgical system for treating tissue comprising:
a microwave signal source (210) capable of providing a microwave frequency output signal;
a microwave amplifier unit (220), wherein the signal source is adapted to provide said output signal to said amplifier unit;
a phase-balanced microwave power splitter (230) having an input port and N output ports, where N is an integer greater than 1, the input port being operatively coupled to the amplifier unit (220); and wherein the system further comprises:
a number N of microwave antenna assemblies (270), each assembly being operatively coupled to the power splitter via a corresponding one of the N output ports,
**characterised in that** each assembly is in the form of a rigid or bendable needle and **in that** the assemblies are arranged in parallel to each other and adapted to be inserted directly into tissue.

2. The system of claim 1, wherein the phase-balanced microwave power splitter (230) provides a substantially equal power split at the N output ports while maintaining a phase balance of <+/-45 degrees.

3. The system of claim 2, further comprising N transmission lines for electrically coupling the N assemblies (270) to the N output ports of the phase-balanced microwave power splitter (230), wherein each transmission line has substantially equal length.

4. The system of claim 3, wherein length is one of electrical length and physical length.

5. The system of claim 4, wherein electrical length is expressed in terms of wavelengths, radians or degrees.

6. The system of claim 1, wherein the phase-balanced microwave power splitter (230) provides a substantially equal power split all output ports while maintaining a phase balance of <+/-10 degrees and an amplitude balance of <1.5 decibels (dB).

## Patentansprüche

1. Elektrochirurgisches System zum Behandeln von Gewebe, mit:
einer Mikrowellensignalquelle (210), die imstande ist, ein Mikrowellenfrequenzausgabesignal bereitzustellen;
einer Mikrowellenverstärkereinheit (220), wobei die Signalquelle angepasst ist, der Verstärkereinheit das Ausgangssignal bereitzustellen;
einem phasenausgeglichenen Mikrowellenleistungsteiler (230), der einen Eingangsanschluss und N Ausgangsanschlüsse aufweist, wobei N eine ganze Zahl größer als 1 ist, der Eingabeanschluss betriebsfähig mit der Verstärkereinheit (220) gekoppelt ist; und
das System ferner aufweist:
eine Anzahl N von Mikrowellenantennenaufbauten (270), wobei jeder Aufbau betriebsfähig mit dem Leistungsteiler über einen entsprechenden der N Ausgabeanschlüsse gekoppelt ist,
**dadurch gekennzeichnet, dass** jeder Aufbau in Form einer steifen oder biegbaren Nadel ist und dass die Aufbauten parallel zueinander angeordnet sind und angepasst sind, direkt in Gewebe eingeführt zu werden.

2. System nach Anspruch 1, bei dem der phasenausgeglichene Mikrowellenleistungsteiler (230) eine im Wesentlichen gleiche Leistungsteilung bei den N Ausgangsanschlüssen bereitstellt, wobei er eine Phasengleichheit von <+/- 45° beibehält.

3. System nach Anspruch 2, ferner mit N Übertragungsleitungen zum elektrischen Koppeln der N Aufbauten (270) mit den N Ausgabeanschlüssen des phasenausgeglichenen Mikrowellenleistungsteilers (230), wobei jede Übertragungsleitung eine im Wesentlichen gleiche Länge aufweist.

4. System nach Anspruch 3, bei dem die Länge eine elektrische Länge oder eine physikalische Länge ist.

5. System nach Anspruch 4, bei dem die elektrische Länge über eine Wellenlänge, einen Radianten oder eine Gradzahl ausgedrückt wird.

6. System nach Anspruch 1, bei dem der phasenausgeglichene Mikrowellenleistungsteiler (230) eine im wesentlichen gleiche Leistungsteilung bei allen Ausgangsanschlüssen bereitstellt, während er eine Phasengleichheit von <+/- 10° und eine Amplitudengleichheit von kleiner 1,5 Dezibel (dB) beibehält.

## Revendications

1. Système électro-chirurgical pour le traitement d'un tissu, comprenant :
une source de signaux micro-ondes (210) apte à fournir un signal de sortie de fréquence micro-onde ;
une unité d'amplification micro-onde (220), où la source de signaux est apte à fournir ledit signal de sortie à ladite unité d'amplification ;
un diviseur de puissance micro-onde équilibré en phase (230) ayant un port d'entrée et N ports de sortie, où N est un entier plus grand que 1, le port d'entrée étant fonctionnellement couplé à l'unité d'amplification (220) ; et où le système comprend en outre :
un nombre N d'ensembles d'antenne micro-onde (270), chaque ensemble étant fonctionnellement couplé au diviseur de puissance par l'intermédiaire d'un des N ports de sortie correspondants,
**caractérisé en ce que** chaque ensemble se présente sous la forme d'une aiguille rigide ou apte à être courbée, et **en ce que** les ensembles sont agencés en parallèle les uns aux autres et sont aptes à être insérés directement dans le tissu.

2. Système selon la revendication 1, dans lequel le diviseur de puissance de micro-onde équilibré en phase (230) fournit une division de puissance sensiblement égale aux N ports de sortie tout en conservant un équilibre des phases de <+/-45 degrés.

3. Système selon la revendication 2, comprenant en outre N lignes de transmission pour coupler électriquement les N ensembles (270) aux N ports de sortie du diviseur de puissance micro-onde équilibré en phase (230), où chaque ligne de transmission a sensiblement une longueur égale.

4. Système selon la revendication 3, dans lequel la longueur est une d'une longueur électrique et d'une longueur physique.

5. Système selon la revendication 4, dans lequel la longueur électrique est exprimée en termes de longueurs d'onde, de radians ou de degrés.

6. Système selon la revendication 1, dans lequel le diviseur de puissance micro-onde équilibré en phase (230) fournit une division de puissance sensiblement égale à tous les ports de sortie tout en conversant un équilibre des phases de <+/-10 degrés et un équilibre d'amplitude de <1,5 décibels (dB).
